# DEMANDE DE BREVET EUROPEEN

(11) **EP 2 460 518 A1**
(43) Date de publication de la demande: **06.06.2012**
(21) Numéro de dépôt: 11191672.2
(22) Date de dépôt: 02.12.2011
(51) Int. Cl.: A61K 31/375, A61K 31/592, A61K 31/593, A61K 31/7008, A61K 31/737, A61P 19/02, A61K 9/20, A61K 9/48, A61K 36/185, A23L 1/00, A61K 8/00

(54) **Composition comprenant un élément chondroprotecteur et des vitamines**

(30) Priorité: 02.12.2010 FR 1060045
(71) Demandeur: SP2L, 84000 Avignon (FR)
(72) Inventeur: Mathez, Laetitia, 13210 Saint-Rémy-de-Provence (FR)
(74) Mandataire: Icosa

(57) **Abrégé**

La présente invention concerne une composition comprenant au moins un élément chondroprotecteur sélectionné parmi le groupe comprenant la glucosamine et ses dérivés et la chondroïtine et ses dérivés, de la vitamine C et de la vitamine D, ladite composition étant utile pour réduire la dégradation du cartilage et/ou accélérer sa régénération chez un sujet.

## Description

### DOMAINE DE L'INVENTION

La présente invention concerne le domaine de la protection du cartilage. La présente invention concerne plus particulièrement une composition comprenant de la chondroïtine et/ou de la glucosamine, de la vitamine C et de la vitamine D, utile pour lutter contre la dégradation du cartilage, et accélérer sa régénération.

### ÉTAT TECHNIQUE ANTÉRIEUR À L'INVENTION

L'arthrose est une maladie fortement invalidante, correspondant à une dégénérescence du cartilage des articulations et à une baisse du volume du cartilage, sans infection ni inflammation particulière. L'arthrose est la maladie articulaire la plus fréquente. Les premiers symptômes apparaissent généralement à partir de 40-50 ans, et se manifestent dans un premier temps par une gêne, puis par de violentes douleurs. Les lésions arthrosiques (fissuration du tissu cartilagineux de la surface vers la profondeur) aboutissent à un raidissement articulaire pouvant évoluer vers une impotence partielle ou totale de l'articulation touchée.

Le cartilage est un tissu conjonctif spécialisé, qui tapisse les articulations et en renforce la solidité. Il est formé de cellules spécialisées, les chondrocytes, et d'une matrice extracellulaire particulière synthétisée par les chondrocytes et composée de glycosaminoglycanes (isolés ou associés à des protéines pour former des protéoglycanes) et de collagène. La synthèse de la matrice extracellulaire par les chondrocytes est lente, le cartilage se développe et se répare donc lentement. A l'âge adulte, il y a peu, voire plus, de régénération du cartilage. La destruction du cartilage, à la suite d'accidents ou d'arthrose, est donc irréversible.

Il existe donc un besoin de solutions pour lutter contre la dégradation du cartilage, ou favoriser sa régénération.

Plusieurs éléments chondroprotecteurs, qui pourraient lutter contre la dégradation du cartilage, notamment liée à l'âge, ont été décrits dans la littérature scientifique.

W0200193832 décrit une composition comprenant de la glucosamine ou l'un de ses sels, par exemple la glucosamine sulfate ou la glucosamine chlorhydrate, pour protéger le cartilage. La glucosamine est le précurseur des glycosaminoglycanes du cartilage. Elle est synthétisée naturellement par le corps humain, mais, avec l'âge, les capacités de synthèse diminuent. De nombreux essais cliniques ont décrit l'effet bénéfique de la glucosamine dans le traitement ou la prévention de l'arthrose.

US 5,364,845 décrit une composition pour protéger, traiter et réparer les tissus conjonctifs tels que le cartilage, et comprenant de la glucosamine en association avec de la chondroïtine sulfate. La chondroïtine est un des glycosaminoglycanes qui composent le cartilage. La chondroïtine pourrait inhiber les enzymes qui dégradent le cartilage, ce qui permettrait de limiter sa dégradation. De même que pour la glucosamine, de nombreuses études scientifiques ont suggéré un effet bénéfique de l'administration de chondroïtine sur la santé du cartilage.

Des études scientifiques récentes semblent montrer un rôle protecteur de la vitamine D sur le cartilage. En effet, ces études révèlent une association entre le taux de vitamine D (et plus particulièrement de 25-hydroxy-vitamine D) et le volume du cartilage. Cet effet pourrait être lié à la fixation de la vitamine D sur ses récepteurs cartilagineux, ce qui stimulerait la production de protéoglycanes et modulerait l'activité des métalloprotéases qui dégradent le cartilage. La vitamine D pourrait également protéger de l'arthrose via un effet sur les os : elle participe en effet au métabolisme osseux, en aidant par exemple à la calcification des os (Ding et al., Arthritis & Rheumatism, 2009, 60(5) :1381-9).

La vitamine C pourrait également avoir un effet chondroprotecteur. D'une part, via son pouvoir antioxydant, la vitamine C pourrait inhiber la dégradation des protéoglycanes et du collagène par les radicaux libres. D'autre part, des résultats expérimentaux *in vitro* démontrent un effet de la vitamine C sur la production de la matrice extracellulaire et notamment des protéoglycanes et du collagène (Clark et al., Matrix Biology, 2002, 21 :175-84). Cependant, une étude sur un modèle d'arthrose du genou *in vivo* chez le cochon d'Inde a démontré un effet néfaste de l'administration de vitamine C sur la progression et l'aggravation de l'arthrose (Kraus *et al.,* 2004, 50(6) :1822-31).

Les compositions de l'art antérieur ont une efficacité limitée, et ne font que freiner la progression de l'arthrose. La Demanderesse s'est alors intéressée à une composition améliorée, permettant d'augmenter l'efficacité de protection du cartilage, à la fois via l'inhibition de sa dégradation et l'augmentation de sa régénération.

De façon surprenante, et contrairement à l'enseignement de l'art antérieur, notamment de Kraus *et al.,* la Demanderesse a identifié un effet synergique des éléments chondroprotecteurs, et particulièrement de l'association de la glucosamine et/ou de la chondroïtine avec les vitamines C et D dans la protection du cartilage. Le terme « effet synergique » tel qu'utilisé dans la présente invention, se réfère à un phénomène dans lequel la combinaison de deux ou plus agents agissant ensemble induit une réponse plus importante que la somme des réponses de chaque agent pris individuellement.

### RÉSUMÉ

La présente invention concerne donc une composition comprenant
- de la vitamine C,
- de la vitamine D et
- au moins un élément chondroprotecteur sélectionné parmi le groupe comprenant la glucosamine et ses dérivés et la chondroïtine sulfate et ses dérivés
   ladite composition ne comprenant pas d'acide hyaluronique ou de collagène.

Selon un mode de réalisation de l'invention, la composition comprend un dérivé de glucosamine, de préférence sélectionné parmi le groupe comprenant la glucosamine sulfate, la glucosamine chlorhydrate et la N-glucosamine. Selon un mode de réalisation de l'invention, la composition est telle que la quantité de glucosamine ou de l'un de ses dérivés administrée par jour varie de 100 à 3000 mg/jour, de préférence de 500 à 2000 mg/jour, plus préférentiellement de 1000 à 1500 mg/jour, encore plus préférentiellement est d'environ 1200 mg/jour ; ou que ladite composition comprend de 50 à 3000 mg de glucosamine ou de l'un de ses dérivés, de préférence de 100 à 2000 mg, plus préférentiellement de 200 à 1750 mg, encore plus préférentiellement de 400 à 1500 mg, encore plus préférentiellement de 600 à 1200 mg, encore plus préférentiellement comprend environ 1200 mg de glucosamine ou de l'un de ses dérivés.

Selon un mode de réalisation de l'invention, la composition comprend de la chondroïtine sulfate. Selon un mode de réalisation, la composition de l'invention est telle que la quantité de chondroïtine sulfate administrée par jour varie de 100 à 3000 mg/jour, de préférence de 300 à 2000 mg/jour, plus préférentiellement de 1000 à 1500 mg/jour, encore plus préférentiellement est d'environ 1200 mg/jour ; ou que ladite composition comprend de 50 à 3000 mg de chondroïtine sulfate, de préférence de 100 à 2000 mg, plus préférentiellement de 200 à 1750 mg, encore plus préférentiellement de 400 à 1500 mg, encore plus préférentiellement de 600 à 1200 mg, encore plus préférentiellement comprend environ 1200 mg de chondroïtine sulfate.

Selon un mode de réalisation de l'invention, la vitamine C présente dans la composition est sélectionnée parmi le groupe comprenant l'acide L-ascorbique, le L-ascorbate de sodium, le L-ascorbate de calcium, le L-ascorbate de potassium, le 6-palmitate de L-ascorbyl, le L-ascorbate de magnésium et le L-ascorbate de zinc. Avantageusement, la composition est telle que la quantité de vitamine C administrée par jour varie de 10 à 2000 mg/jour, de préférence de 90 à 1000 mg/jour, plus préférentiellement de 150 à 500 mg/jour, encore plus préférentiellement de 160 à 250 mg/jour, encore plus préférentiellement est d'environ 180 mg/jour; ou que ladite composition comprend de 30 à 2000 mg de vitamine C, de préférence de 60 à 1000 mg, plus préférentiellement de 90 à 500 mg, encore plus préférentiellement de 180 à 250 mg, encore plus préférentiellement comprend environ 180 mg de vitamine C.

Selon un mode de réalisation de l'invention, la vitamine D présente dans la composition est sélectionnée parmi le groupe comprenant le cholécalciférol, l'ergocalciférol et leurs dérivés conservant la même activité. Avantageusement, la composition est telle que la quantité de vitamine D administrée par jour varie de 0.5 à 50 µg/jour, de préférence de 1 à 25 µg/jour, plus préférentiellement de 2.5 à 15 µg/jour, encore plus préférentiellement de 5 à 10 µg/jour, encore plus préférentiellement est d'environ 5 µg/jour; ou que ladite composition comprend de 0.5 à 50 µg de vitamine D, de préférence de 1 à 25 µg, plus préférentiellement de 2.5 à 15 µg, encore plus préférentiellement de 5 à 10 µg, encore plus préférentiellement est d'environ 5 µg de vitamine D.

Selon un mode de réalisation de l'invention, la composition est un produit nutraceutique, un produit alimentaire, une boisson, un complément alimentaire, un alicament, un produit cosmétique, un produit d'alimentation vétérinaire ou un médicament.

Selon un mode de réalisation de l'invention, la composition peut être administrée par voie orale, par voie buccale ou par voie transcutanée.

Selon un mode de réalisation de l'invention, la composition est sous la forme de granules, comprimés, capsules souples, poudres, solutions pour injection, crèmes, gels, émulsions, onguents, lavements, suspensions, sirops, ampoules, inhalateurs, sprays pour la bouche, injections, gouttes, suppositoires, patches, patchs transdermiques, pâtes, pommades, gommes à mâcher, poudres à dissoudre, solutions ou gélules.

Selon un mode de réalisation de l'invention, la composition est utile pour réduire la dégradation du cartilage et/ou accélérer sa régénération chez un sujet.

L'invention concerne également une composition telle que décrite ci-dessus pour le traitement de la douleur articulaire.

L'invention concerne également une composition telle que décrite ci-dessus pour le traitement de l'arthrose.

### DESCRIPTION DÉTAILLÉE

La présente invention concerne donc une composition comprenant :
- au moins un élément chondroprotecteur sélectionné parmi le groupe comprenant la glucosamine et ses dérivés et la chondroïtine sulfate et ses dérivés,
- de la vitamine C et
- de la vitamine D.

La présente invention concerne également une composition consistant en :
- au moins un élément chondroprotecteur sélectionné parmi le groupe comprenant la glucosamine et ses dérivés et la chondroïtine et ses dérivés,
- de la vitamine C et
- de la vitamine D.

Selon un mode de réalisation de l'invention, la composition comprend de la glucosamine ou l'un de ses dérivés. La glucosamine est un monosaccharide aminé, de formule (2-amino-2-déoxy-D-Glucose).

Avantageusement, la composition comprend un dérivé de la glucosamine, de préférence sélectionné parmi le groupe comprenant la glucosamine sulfate (stabilisée par le chlorure de sodium ou le chlorure de potassium), la glucosamine sulfate dipotassique, la glucosamine chlorhydrate et la N-acétylglucosamine. Encore plus préférentiellement, la composition comprend la glucosamine sulfate.

La glucosamine ou l'un de ses dérivés sont commercialement disponibles, par exemple chez GEE LAWSON. De préférence, la glucosamine ou l'un de ses dérivés est la Glucosamine sulfate, 2 KCI et provient de chez GEE LAWSON, référence SC2000155.

Selon un mode de réalisation de l'invention, la composition est telle que la quantité de glucosamine ou de l'un de ses dérivés administrée par jour varie de 100 à 3000 mg/jour, de préférence de 500 à 2000 mg/jour, plus préférentiellement de 1000 à 1500 mg/jour, encore plus préférentiellement est d'environ 1200 mg/jour. Selon un mode de réalisation de l'invention, la quantité de glucosamine ou de l'un de ses dérivés est comprise entre environ 500, 550, 600, 650, 700, 750, 800, 850, 900, 910, 920, 930, 940, 950, 960, 970, 980, 990, 1000, 1010, 1020, 1030, 1040, 1050, 1060, 1070, 1080, 1090, 1100, 1110, 1120, 1130, 1140, 1150, 1160, 1170, 1180, 1190, 1200, 1250, 1300, 1350, 1400, 1450, 1500, 1600, 1700, 1800, 1900 et 2000 mg/jour.

Selon un mode de réalisation de l'invention, la composition comprend de 50 à 3000 mg, de glucosamine ou de l'un de ses dérivés, de préférence de 100 à 2000 mg, plus préférentiellement de 200 à 1750 mg, encore plus préférentiellement de 400 à 1500 mg, encore plus préférentiellement de 600 à 1200 mg, encore plus préférentiellement comprend environ 1200 mg de glucosamine ou de l'un de ses dérivés. Selon un mode de réalisation de l'invention, la composition comprend de 50 à 3000 mg de glucosamine ou de l'un de ses dérivés, de préférence de 100 à 2000 mg, plus préférentiellement de 200 à 1500 mg, encore plus préférentiellement de 300 à 1100 mg. Selon un mode de réalisation de l'invention, la composition est comprise entre environ 140 mg de glucosamine ou de l'un de ses dérivés, 150, 160, 170, 180, 190, 200, 210, 220, 230, 240, 250, 260, 270, 280,2 90, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 750, 800, 850, 900, 950, 1000, 1050, 1100, 1150, 1200, 1250, 1300, 1350, 1400, 1450 et 1500 mg.

Selon l'invention, par « environ », on entend, lorsque ce terme est placé devant une valeur numérique, jusqu'à 10% en plus ou en moins de ladite valeur numérique.

Selon un mode de réalisation de l'invention, la composition comprend de la chondroïtine sulfate, ou l'un de ses dérivés. Par dérivés de la chondroïtine sulfate, on entend l'un de ses sels, tel que, par exemple, le sel de sodium de la chondroïtine sulfate. La chondroïtine sulfate est un glycosaminoglycane constitué d'une chaîne de taille variable d'acide D-glucuronique et de N-acétyl-D-galactosamine-4/6-sulfate.

La chondroïtine sulfate est commercialement disponible, par exemple chez BIOSERAE. De préférence, la chondroïtine sulfate provient de chez BIOSERAE, référence 410158.

Selon un mode de réalisation de l'invention, la composition est telle que la quantité de chondroïtine sulfate administrée par jour varie de 100 à 3000 mg/jour, de préférence de 300 à 2000 mg/jour, plus préférentiellement de 1000 à 1500 mg/jour, encore plus préférentiellement est d'environ 1200 mg/jour. Selon un mode de réalisation de l'invention, la composition est telle que la quantité de chondroïtine sulfate administrée par jour varie de 100 à 1000 mg/jour, de préférence de 150 à 700 mg/jour, plus préférentiellement de 200 à 500 mg/jour, encore plus préférentiellement est d'environ 240 mg/jour. Selon un mode de réalisation de l'invention, la composition est telle que la quantité de chondroïtine sulfate administrée par jour est comprise entre environ 150 mg/jour, 160, 170, 180, 190,200,210,220,230,240,250,260,270,280,290,300,310, 320, 330, 340, 350, 400, 450, 500, 550, 600, 650, 700, 750, 800, 850, 900, 925, 950, 975, 1000, 1025, 1050, 1075, 1100,1125, 1150, 1175, 1200, 1250, 1300, 1350, 1400, 1450, 1500, 1600, 1700, 1800, 1900 et 2000 mg/jour.

Selon un mode de réalisation de l'invention, la composition comprend de 50 à 3000 mg de chondroïtine sulfate, de préférence de 100 à 2000 mg, plus préférentiellement de 200 à 1750 mg, encore plus préférentiellement de 400 à 1500 mg, encore plus préférentiellement de 600 à 1200 mg, encore plus préférentiellement comprend environ 1200 mg de chondroïtine sulfate. Selon un mode de réalisation de l'invention, la composition comprend de 10 à 500 mg de chondroïtine sulfate, de préférence de 50 à 250 mg, encore plus préférentiellement de 80 à 240 mg. Selon un mode de réalisation de l'invention, la composition comprend entre environ 50 mg de chondroïtine sulfate, 60, 70, 80, 90, 100, 110, 120, 130, 140, 150, 160, 170, 180, 190, 200, 210, 220, 230, 240, 250, 260, 270, 280,2 90, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 750, 800, 850, 900, 950, 1000, 1050, 1100, 1150, 1200, 1250, 1300, 1350, 1400, 1450 et 1500 mg.

Selon un mode de réalisation, la vitamine C présente dans la composition selon l'invention est sélectionnée dans le groupe comprenant l'acide L-ascorbique, L-ascorbate de sodium, L-ascorbate de calcium, L-ascorbate de potassium, 6-palmitate de L-ascorbyl, L-ascorbate de magnésium, L-ascorbate de zinc, conformément au règlement (CE) n°1170/2009 de la Commission du 30 novembre 2009. De préférence, la vitamine C présente dans la composition est de l'acide ascorbique.

La vitamine C est commercialement disponible, par exemple chez CSPC WEIHENG PHARMACEUTICAL. De préférence, la vitamine C est de l'acide ascorbique et provient de chez CSPC WEIHENG PHARMACEUTICAL, référence « Ascorbic acid EP».

Selon un mode de réalisation de l'invention, la composition est telle que la quantité de vitamine C administrée par jour varie de 50 à 2000 mg/jour, de préférence de 90 à 1000 mg/jour, plus préférentiellement de 150 à 500 mg/jour, encore plus préférentiellement de 160 à 250 mg/jour, encore plus préférentiellement est d'environ 180 mg/jour. Selon un mode de réalisation de l'invention, la composition est telle que la quantité de vitamine C administrée par jour varie de 10 à 500 mg/jour, de préférence de 25 à 250 mg/jour, plus préférentiellement de 50 à 100 mg/jour, encore plus préférentiellement est d'environ 80 mg/jour. Selon un mode de réalisation de l'invention, la composition est telle que la quantité de vitamine C administrée par jour est comprise entre environ 20 mg/jour, 30, 40, 50, 60, 70, 80, 90, 100, 110, 120, 130, 140, 150, 160, 170, 180, 190, 200, 210, 220, 230, 240, 250, 300, 350, 400, 450 et 500 mg/jour.

Selon un mode de réalisation de l'invention, la composition comprend de 30 à 2000 mg de vitamine C, de préférence de 60 à 1000 mg, plus préférentiellement de 90 à 500 mg, encore plus préférentiellement de 180 à 250 mg, encore plus préférentiellement comprend environ 180 mg de vitamine C. Selon un mode de réalisation de l'invention, la composition comprend de 3 à 500 mg de vitamine C, de préférence de 8 à 250 mg, plus préférentiellement de 15 à 100 mg, encore plus préférentiellement de 25 à 80 mg de vitamine C. Selon un mode de réalisation de l'invention, la composition comprend entre environ 10 mg de vitamine C, 20, 25, 30, 35, 40, 45, 50, 55, 60, 65, 70, 75, 80, 85, 90,95,100,110,120,130,140,150,160,170, 180, 190, 200, 210, 220, 230, 240, 250, 300, 350, 400, 450 et 500 mg.

La vitamine D est une vitamine liposoluble synthétisée par l'organisme humain à partir d'un dérivé du cholestérol sous l'action des rayonnements UVB de la lumière. Elle existe sous deux formes : la vitamine D2 (ergocalciférol) et la vitamine D3 (cholécalciférol). Selon un mode de réalisation, la vitamine D présente dans la composition selon l'invention est sélectionnée dans le groupe comprenant Cholécalciférol, Ergocalciférol, et dérivés conservant la même activité, tels que, par exemple la 25-hydroxyvitamine D, la 1,25-dihydroxyvitamine D et la 24,25-dihydroxyvitamine D. De préférence, la vitamine D présente dans la composition est le Cholécalciférol ou l'Ergocalciférol.

La vitamine D est commercialement disponible, par exemple chez LYCORED. De préférence, la vitamine D est de la vitamine D3, disponible sous la dénomination CapsuDar®D3 100CWD chez LYCORED, référence 08150.

Selon un mode de réalisation de l'invention, la composition est telle que la quantité de vitamine D administrée par jour varie de 0.5 à 50 µg/jour, de préférence de 1 à 25 µg/jour, plus préférentiellement de 2.5 à 15 µg/jour, encore plus préférentiellement de 5 à 10 µg/jour, encore plus préférentiellement est d'environ 5 µg/jour. Selon un mode de réalisation de l'invention, la composition est telle que la quantité de vitamine D administrée par jour est comprise entre environ 0.5 µg/jour, 1, 1.5, 2, 2.5, 3, 3.5, 4, 4.5, 5, 5.5, 6, 6.5, 7, 7.5, 8, 8.5, 9, 9.5, 10, 11, 12, 13, 14, 15, 20, 25, 30, 40 et 50 µg/jour.

Selon un mode de réalisation de l'invention, la composition comprend de 0.5 à 50 µg de vitamine D, de préférence de 1 à 25 µg, plus préférentiellement de 2.5 à 15 µg, encore plus préférentiellement de 5 à 10 µg, encore plus préférentiellement est d'environ 5 µg de vitamine D. Selon un mode de réalisation de l'invention, la composition comprend entre environ 0.5 µg de vitamine D, 1, 1.5, 2, 2.5, 3, 3.5, 4, 4.5, 5, 5.5, 6, 6.5, 7, 7.5, 8, 8.5,9,9.5, 10, 11, 12, 13, 14, 15, 20, 25, 30, 40 et 50 µg.

Selon un mode de réalisation de l'invention, la composition comprend de la glucosamine ou l'un de ses dérivés, de la vitamine C et de la vitamine D.

Selon un mode de réalisation de l'invention, la composition est telle que :
■ la quantité de glucosamine ou de l'un de ses dérivés administrée par jour varie de 100 à 3000 mg/jour, de préférence de 500 à 2000 mg/jour, plus préférentiellement de 1000 à 1500 mg/jour, encore plus préférentiellement est d'environ 1200 mg/jour ; et
■ la quantité de vitamine C administrée par jour varie de 10 à 2000 mg/jour, de préférence de 50 à 2000 mg/jour, de préférence de 90 à 1000 mg/jour, plus préférentiellement de 150 à 500 mg/jour, encore plus préférentiellement de 160 à 250 mg/jour, encore plus préférentiellement est d'environ 180 mg/jour ; et
■ la quantité de vitamine D administrée par jour varie de 0.5 à 50 µg/jour, de préférence de 1 à 25 µg/jour, plus préférentiellement de 2.5 à 15 µg/jour, encore plus préférentiellement de 5 à 10 µg/jour, encore plus préférentiellement est d'environ 5 µg/jour.

Selon un mode de réalisation de l'invention, la composition comprend :
■ de 50 à 3000 mg, de glucosamine ou de l'un de ses dérivés, de préférence de 100 à 2000 mg, plus préférentiellement de 200 à 1750 mg, encore plus préférentiellement de 400 à 1500 mg, encore plus préférentiellement de 600 à 1200 mg, encore plus préférentiellement comprend environ 1200 mg de glucosamine ou de l'un de ses dérivés ; et
■ de 3 à 2000 mg de vitamine C, de préférence de 30 à 2000 mg de vitamine C, de préférence de 60 à 1000 mg, plus préférentiellement de 90 à 500 mg, encore plus préférentiellement de 180 à 250 mg, encore plus préférentiellement comprend environ 180 mg de vitamine C ; et
■ de 0.5 à 50 µg de vitamine D, de préférence de 1 à 25 µg, plus préférentiellement de 2.5 à 15 µg, encore plus préférentiellement de 5 à 10 µg, encore plus préférentiellement est d'environ 5 µg de vitamine D.

Selon un mode de réalisation de l'invention, la composition comprend de la chondroïtine sulfate, de la vitamine C et de la vitamine D.

Selon un mode de réalisation de l'invention, la composition est telle que :
■ la quantité de chondroïtine sulfate administrée par jour varie de 100 à 3000 mg/jour, de préférence de 300 à 2000 mg/jour, plus préférentiellement de 1000 à 1500 mg/jour, encore plus préférentiellement est d'environ 1200 mg/jour ; et
■ la quantité de vitamine C administrée par jour varie de 10 à 2000 mg/jour, de préférence de 50 à 2000 mg/jour, de préférence de 90 à 1000 mg/jour, plus préférentiellement de 150 à 500 mg/jour, encore plus préférentiellement de 160 à 250 mg/jour, encore plus préférentiellement est d'environ 180 mg/jour ; et
■ la quantité de vitamine D administrée par jour varie de 0.5 à 50 µg/jour, de préférence de 1 à 25 µg/jour, plus préférentiellement de 2.5 à 15 µg/jour, encore plus préférentiellement de 5 à 10 µg/jour, encore plus préférentiellement est d'environ 5 µg/jour.

Selon un mode de réalisation de l'invention, la composition comprend :
■ de 50 à 3000 mg de chondroïtine sulfate, de préférence de 100 à 2000 mg, plus préférentiellement de 200 à 1750 mg, encore plus préférentiellement de 400 à 1500 mg, encore plus préférentiellement de 600 à 1200 mg, encore plus préférentiellement comprend environ 1200 mg de chondroïtine sulfate ; et
■ de 3 à 2000 mg de vitamine C, de préférence de 30 à 2000 mg de vitamine C, de préférence de 60 à 1000 mg, plus préférentiellement de 90 à 500 mg, encore plus préférentiellement de 180 à 250 mg, encore plus préférentiellement comprend environ 180 mg de vitamine C ; et
■ de 0.5 à 50 µg de vitamine D, de préférence de 1 à 25 µg, plus préférentiellement de 2.5 à 15 µg, encore plus préférentiellement de 5 à 10 µg, encore plus préférentiellement est d'environ 5 µg de vitamine D.

Selon un mode de réalisation de l'invention, la composition comprend de la glucosamine ou l'un de ses dérivés, de la chondroïtine sulfate, de la vitamine C et de la vitamine D.

Selon un mode de réalisation de l'invention, la composition est telle que :
■ la quantité de glucosamine ou de l'un de ses dérivés administrée par jour varie de 100 à 3000 mg/jour, de préférence de 500 à 2000 mg/jour, plus préférentiellement de 1000 à 1500 mg/jour, encore plus préférentiellement est d'environ 1200 mg/jour; et
■ la quantité de chondroïtine sulfate administrée par jour varie de 100 à 3000 mg/jour, de préférence de 300 à 2000 mg/jour, plus préférentiellement de 1000 à 1500 mg/jour, encore plus préférentiellement est d'environ 1200 mg/jour ; et
■ la quantité de vitamine C administrée par jour varie de 10 à 2000 mg/jour, de préférence de 50 à 2000 mg/jour, de préférence de 90 à 1000 mg/jour, plus préférentiellement de 150 à 500 mg/jour, encore plus préférentiellement de 160 à 250 mg/jour, encore plus préférentiellement est d'environ 180 mg/jour ; et
■ la quantité de vitamine D administrée par jour varie de 0.5 à 50 µg/jour, de préférence de 1 à 25 µg/jour, plus préférentiellement de 2.5 à 15 µg/jour, encore plus préférentiellement de 5 à 10 µg/jour, encore plus préférentiellement est d'environ 5 µg/jour.

Selon un mode de réalisation de l'invention, la composition comprend :
■ de 50 à 3000 mg, de glucosamine ou de l'un de ses dérivés, de préférence de 100 à 2000 mg, plus préférentiellement de 200 à 1750 mg, encore plus préférentiellement de 400 à 1500 mg, encore plus préférentiellement de 600 à 1200 mg, encore plus préférentiellement comprend environ 1200 mg de glucosamine ou de l'un de ses dérivés ; et
■ de 50 à 3000 mg de chondroïtine sulfate, de préférence de 100 à 2000 mg, plus préférentiellement de 200 à 1750 mg, encore plus préférentiellement de 400 à 1500 mg, encore plus préférentiellement de 600 à 1200 mg, encore plus préférentiellement comprend environ 1200 mg de chondroïtine sulfate ; et

■ de 3 à 2000 mg de vitamine C, de préférence de 30 à 2000 mg de vitamine C, de préférence de 60 à 1000 mg, plus préférentiellement de 90 à 500 mg, encore plus préférentiellement de 180 à 250 mg, encore plus préférentiellement comprend environ 180 mg de vitamine C ; et
■ de 0.5 à 50 µg de vitamine D, de préférence de 1 à 25 µg, plus préférentiellement de 2.5 à 15 µg, encore plus préférentiellement de 5 à 10 µg, encore plus préférentiellement est d'environ 5 µg de vitamine D.

Selon un mode de réalisation de l'invention, chacune des compositions présentées ci-dessus peut comprendre en outre de l'Harpagophytum ou de l'harpagoside.

L'Harpagophytum est une plante de la famille des Pedaliaceae, dont la principale espèce est *Harpagophytum procumbens* communément appelée « griffe du diable » ou « racine de Windhoek ». Une autre espèce connue d'Harpagophytum est l'*Harpagophytum zeyheri.* L'Harpagophytum est une herbacée vivace originaire des régions semi-désertiques sud africaines.

L'Harpagophytum est une plante médicinale dont la racine est inscrite dans la pharmacopée européenne pour le traitement de douleurs articulaires mineures. Les deux principes actifs de l'Harpagophytum sont l'harpagoside et le beta-sitostérol. Ces deux molécules possèdent des propriétés anti-inflammatoires. Les racines d'Harpagophytum contiennent également des flavonoïdes, des acides phénoliques, des quinones, des phytostérols, des sucres, des triterpènes et des acétosides.

Lors de ses recherches, la Demanderesse a identifié que l'ajout d'Harpagophytum ou d'harpagoside à la composition de l'invention présentait un avantage pour lutter contre la dégradation du cartilage, accélérer sa régénération et traiter l'inflammation liée aux affections du cartilage, telles que, par exemple, les rhumatismes, car les éléments chondroprotecteurs, les vitamines et l'Harpagophytum ou l'harpagoside agissent en synergie.

Dans un mode de réalisation de l'invention, la composition selon l'invention comprend l'Harpagophytum sous forme de poudre ou sous forme d'extrait. Les racines secondaires d'Harpagophytum peuvent être utilisées pharmaceutiquement et peuvent être préparées sous forme de thé, sous forme de poudre ou subir une extraction pour l'obtention d'un extrait sec ou liquide.

De manière générale, les extraits d'Harpagophytum comprennent 0,5 à 20% d'harpagoside, mesuré par HPLC. Ces extraits offrent une alternative satisfaisante à l'usage de poudre de plante.

Le procédé décrit dans W02008/145931 permet d'obtenir des extraits d'Harpagophytum sous forme liquide ou sèche, comprenant une concentration en harpagoside supérieur ou égal à 5%, voire supérieur ou égal à 35%.

Des extraits susceptibles d'être utilisés dans la présente invention sont par exemple les extraits commercialisés par Naturex sous le nom de Devil's Claw, ou les extraits commercialisés par Burgundy sous le nom Botany Harpagophytum (5% harpagoside) ou BotanySelect Harpagophytum (20% harpagoside).

Selon un mode de réalisation de l'invention, la composition est telle que la quantité de poudre d'Harpagophytum administrée par jour varie de 100 à 3000 mg/jour, de préférence de 200 à 2000 mg/jour, plus préférentiellement de 400 à 1500 mg/jour, encore plus préférentiellement de 600 à 900 mg/jour, encore plus préférentiellement est d'environ 800 mg/jour.

Selon un mode de réalisation de l'invention, la composition comprend de 30 à 3000 mg de poudre d'Harpagophytum, de préférence de 100 à 2000 mg, plus préférentiellement de 250 à 1500 mg, encore plus préférentiellement de 400 à 1000 mg, encore plus préférentiellement est d'environ 65 mg de poudre d'Harpagophytum.

Dans un mode de réalisation de l'invention, la composition est telle que la quantité d'extrait d'Harpagophytum contenant 0,5 à 5% d'harpagosides administrée par jour varie de 50 à 3000 mg/jour, de préférence 100 à 1000 mg/jour, plus préférentiellement de 150 à 700 mg/jour, encore plus préférentiellement de 200 à 500 mg/jour. Dans un mode de réalisation de l'invention, la composition est telle que la quantité d'extrait d'Harpagophytum contenant 0,5 à 5% d'harpagosides administrée par jour est d'environ 400 mg d'extrait d'Harpagophytum contenant 0,5 à 5% d'harpagosides par jour. Dans un autre mode de réalisation de l'invention, la composition selon l'invention est telle que la quantité d'extrait d'Harpagophytum contenant 0,5 à 5% d'harpagosides administrée par jour est d'environ 250 mg.

Dans un mode de réalisation de l'invention, la composition comprend de 50 à 3000 mg d'extrait d'Harpagophytum contenant 0,5 à 5% d'harpagosides, de préférence 100 à 1000 mg, plus préférentiellement de 150 à 700 mg, encore plus préférentiellement de 200 à 500 mg. Dans un mode de réalisation, la composition selon l'invention comprend 400 mg d'extrait d'Harpagophytum contenant 0,5 à 5% d'harpagosides. Dans un autre mode de réalisation, la composition selon l'invention comprend 250 mg d'extrait d'Harpagophytum contenant 0,5 à 5% d'harpagosides.

Dans un mode de réalisation de l'invention, la composition est telle que la quantité d'harpagosides administrée par jour varie de 0.1 à 200 mg/jour, de préférence de 1 à 100 mg/jour, de préférence 10 à 50 mg/jour, plus préférentiellement de 15 à 30 mg/jour et encore plus préférentiellement 20 mg d'harpagoside par jour.

Dans un mode de réalisation de l'invention, la composition comprend de 0.1 à 200 mg d'harpagosides, de préférence de 0.3 à 100 mg d'harpagosides, de préférence 5 à 50 mg, plus préférentiellement de 10 à 30 mg et encore plus préférentiellement 20 mg d'harpagoside.

Selon un mode de réalisation de l'invention, la composition ne comprend pas de collagène.

Selon un mode de réalisation de l'invention, la composition ne comprend pas d'acide hyaluronique.

Selon un mode de réalisation de l'invention, la composition ne comprend pas de vitamine K.

Selon un mode de réalisation de l'invention, la composition ne comprend pas de manganèse, ou l'un de ses dérivés, tel que, par exemple, l'ascorbate de manganèse.

Selon un mode de réalisation de l'invention, tous les éléments qui composent la composition sont contenus dans une forme galénique unique.

Selon un mode de réalisation de l'invention, la composition selon l'invention peut être un produit nutraceutique, un produit alimentaire, une boisson, un complément alimentaire, un alicament, un produit cosmétique, un produit d'alimentation vétérinaire ou un médicament. Par « produit nutraceutique », on entend un produit isolé ou purifié à partir d'un élément comestible, ayant un effet physiologique prouvé bénéfique ou protecteur contre un désordre ou un inconfort. Par « complément alimentaire », on entend un produit comprenant des vitamines, minéraux, végétaux, acides aminés, concentrés, métabolites, constituants, extraits ou une combinaison de ces ingrédients. Par « alicament », ou aliment fonctionnel, on entend un aliment modifié, ou un ingrédient alimentaire modifié ayant un effet bénéfique ou protecteur contre un désordre ou un inconfort, au-delà des nutriments qu'il contient.

Selon un mode de réalisation de l'invention, la composition selon l'invention comprend optionnellement un ou plusieurs excipients pharmaceutiquement ou nutraceutiquement acceptables ou des sels ou des additifs.

Selon un mode de réalisation de l'invention, la composition comprend en outre un ou plusieurs additifs, sélectionnés dans le groupe comprenant les agents de charge (diluant, liants, délitant), les antiagglomérants, les agents émulsionnants, les agents colorants et les agents sucrants.

Dans un mode de réalisation de l'invention, la composition selon l'invention est formulée sous une forme de dosage solide, semi-solide ou liquide par addition de véhicules biologiquement ou pharmaceutiquement ou nutraceutiquement acceptables.

Des exemples de véhicules biologiquement ou pharmaceutiquement ou nutraceutiquement acceptables incluent, mais ne sont pas limités à, des surfactants, des excipients, des liants, des diluants, des lubrifiants, des conservateurs, des stabilisateurs, des antioxygènes, des tampons, des suspensions et des systèmes d'administration.

Des exemples de véhicules liquides incluent de l'eau distillée, une solution saline, une solution aqueuse de glucose, de l'alcool par exemple de l'éthanol, du propylène glycol, et du polyéthylène glycol ; et des véhicules huileux tels que des huiles végétales et animales, de la paraffine, ou de la cire.

Des exemples de véhicules, diluants ou excipients solides incluent, mais ne sont pas limités au glucose, fructose, sucrose, maltose, dextrine jaune, dextrine blanche, maltodextrine, cellulose microcristalline, stéarate de calcium, stéarate de magnésium, sorbitol, sirop de glucose, lactose, acide citrique, acide tartrique, acide malique, acide succinique, acide lactique, acide L-ascorbique, alpha-tocophérol, glycérol, propylène glycol, le sucroester, les esters poly glycériques d'acides gras, les sucroglycérides, les mono, di et triglycérides béhénate, les Carraghénanes, la gomme arabique, la caséine, la gélatine, la pectine, l'agar, la nicotinamide, les acides aminés, les sels de calciums, les pigments...

Des exemples d'antioxygénes incluent mais ne sont pas limités à du tocophérol, du butylhydroxytoluène (BHT), du butylhydroxyanisol (BHA), des antioxydants naturels comme la vitamine E, l'extrait de romarin, du gallate de propyle...

Des exemples de conservateurs antimicrobiens incluent mais ne sont pas limités à du méthylparabène, du Propylparaben, du sorbate de potassium, du benzoate de sodium, de l'acide benzoïque...

Des exemples d'antiagglomérants incluent mais ne sont pas limités à du dioxyde de silicium.

Des exemples de surfactants incluent mais ne sont pas limités à des surfactants anioniques, cationiques, ou non-ioniques tels que le palmitate d'ascorbyle, les polysorbates, les polyéthylènes glycols...

Des exemples d'agents stabilisateurs du pH ou des tampons incluent mais ne sont pas limités à acide citrique-citrate de sodium, acide phosphorique-phosphate de sodium, acide acétique-acétate de sodium...

La composition selon l'invention est administrée de préférence par voie entérale ou par voie topique. Une administration parentérale peut également être envisagée. On entend par voie entérale la voie orale, la voie buccale, la voie sublinguale, la voie rectale, la voie pulmonaire, la voie percutanée et les voies locales.

Selon un mode de réalisation de l'invention, la composition est sous une forme adaptée à une administration orale. On entend par « administration orale » une administration dans la cavité buccale, suivie par l'ingestion d'un composé, qui rejoint la circulation systémique à la suite de son absorption intestinale. Selon un mode de réalisation de l'invention, la composition est sous une forme solide, telle que, par exemple, des granules, une poudre, une gélule, un comprimé ou une capsule souple. Selon un autre mode de réalisation de l'invention, la composition est sous forme liquide, telle que, par exemple, une solution, une suspension, une poudre à dissoudre, une boisson ou un sirop.

Selon un mode de réalisation de l'invention, la composition est sous une forme adaptée à une administration buccale, telle que par exemple une gomme à mâcher, un patch ou un spray buccal. On entend par « administration buccale » une administration dans la cavité buccale d'un composé, qui n'est pas suivie de l'ingestion dudit composé, dont l'absorption se fait à travers les tissus buccaux, tels que, par exemple, le palais, le tissu sublingual, ou les gencives.

Selon un mode de réalisation de l'invention, la composition est sous une forme adaptée à une administration transcutanée, telle que, par exemple, une pommade, une pâte, un onguent, un gel, une crème ou un patch transdermique. Par « administration cutanée », ou « administration transdermique », on entend l'administration d'un composé sur la peau, suivie de son absorption dans la circulation sanguine systémique à travers les tissus cutanés adjacents.

Dans un mode de réalisation de l'invention, des formes de dosage spécifiques pour la formulation de la composition selon l'invention incluent, mais ne sont pas limitées à, des formulations orales telles que des granules, des comprimés, des gélules, des capsules souples, des poudres, des poudres à dissoudre, des solutions pour injection, des solutions, des suspensions, des crèmes, des gels, des pommades, des pâtes, des émulsions (émulsion huile dans eau, eau dans huile, anhydre, solide ou microémulsions), des onguents, des lavements, des suspensions, des sirops, des ampoules, des gommes à mâcher, des inhalateurs, des sprays pour la bouche, des injections, des gouttes, des suppositoires, des patches, des patchs transdermiques...

Dans un autre mode de réalisation de l'invention, les compositions selon l'invention sont administrées sous forme de comprimés à libération contrôlée, utilisant des revêtements à base de polymères permettant la libération contrôlée grâce à des techniques bien connues de l'homme du métier telles que la micro-encapsulation ou des systèmes de véhicule colloïdal.

Des exemples d'agents d'encapsulation incluent, mais ne sont pas limités à, de l'amidon, des protéines de source animale comme, par exemple, la gélatine, des protéines de source végétale, de la caséine, de la pectine, de l'alginate, de l'agar, des maltodextrines, des sulfonates de lignine, des dérivés cellulosiques (éthylcellulose, méthylcellulose, hydroxypropylcellulose, hydroxypropylméthylcellulose, carboxyméthylcellulose), des sucres, sorbitols, gommes....

Dans un mode de réalisation de l'invention, la composition selon l'invention peut être comprise dans des produits alimentaires ou des boissons tels que du thé, des sucreries, des produits à usage culinaire, des compléments nutritionnels, des produits laitiers, des boissons, des boissons à base de lait, des soupes, des substituts de repas, des barres nutritionnelles, des poudres à base de lait, des produits céréaliers, des biscuits, des gommes à mâcher, du chocolat...

Selon un mode de réalisation de l'invention, la composition est utile pour réduire la dégradation du cartilage et/ou accélérer sa régénération chez un sujet. Avantageusement, la composition selon l'invention est utile pour réduire la douleur et la raideur articulaire liées à une affection du cartilage, telle que, par exemple, les rhumatismes.

La présente invention concerne également l'utilisation de la composition telle que décrite ci-dessus pour réduire la dégradation du cartilage et/ou accélérer sa régénération chez un sujet. Selon un mode de réalisation, on utilise la composition de l'invention pour réduire la douleur et la raideur articulaire liées à une affection du cartilage, telle que, par exemple, les rhumatismes. Selon un mode de réalisation, la composition selon l'invention est utile pour le traitement des rhumatismes et/ou de l'arthrose.

La présente invention concerne également une méthode pour réduire la dégradation du cartilage et/ou accélérer sa régénération, comprenant l'administration de la composition telle que décrite ci-dessus chez un sujet. Selon un mode de réalisation, la méthode selon l'invention a pour but de réduire la douleur et la raideur articulaire liées à une affection du cartilage, telle que, par exemple, les rhumatismes.

La présente invention concerne également une composition telle que décrite précédemment pour traiter la douleur articulaire. La présente invention concerne également une composition telle que décrite précédemment pour réduire la douleur articulaire chez un sujet. Selon un mode de réalisation de l'invention, la douleur articulaire est liée à une affection du cartilage, telle que, par exemple, les rhumatismes ou l'arthrose.

La présente invention concerne également une composition telle que décrite ci-dessus pour le traitement des rhumatismes et/ou de l'arthrose.

Selon un mode de réalisation de l'invention, la composition est administrée oralement. Selon un autre mode de réalisation de l'invention, la composition est administrée de façon buccale. Selon un autre mode de réalisation de l'invention, la composition est administrée de façon transdermique.

Selon un mode de réalisation de l'invention, la composition est utile pour lutter contre la dégradation et/ou accélérer la régénération du cartilage hyalin articulaire ou non-articulaire, de préférence du cartilage hyalin articulaire, ou pour traiter les douleurs liées à la dégradation du cartilage hyalin articulaire ou non-articulaire, de préférence du cartilage hyalin articulaire. Le cartilage hyalin articulaire se trouve à la surface des articulations mobiles de type synovial (i.e. avec une capsule synoviale). Le cartilage hyalin non-articulaire se trouve, par exemple, dans la cloison nasale, dans les cartilages thyroïdes et cricoïdes, dans les anneaux des grosses bronches et de la trachée, et à l'extrémité des côtes.

Selon un autre mode de réalisation de l'invention, la composition est utile pour lutter contre la dégradation et/ou accélérer la régénération du cartilage fibreux, ou pour traiter les douleurs liées à la dégradation du cartilage fibreux. Le cartilage fibreux, ou fibrocartilage, se trouve, par exemple, dans les disques intervertébraux, dans la symphyse pubienne, dans les ménisques des genoux et dans les sites d'insertion des ligaments, tendons et capsules articulaires.

Selon un mode de réalisation de l'invention, la composition est utile pour lutter contre la dégradation et/ou accélérer la régénération du cartilage élastique, ou pour traiter les douleurs liées à la dégradation du cartilage élastique. Le cartilage élastique se trouve, par exemple, dans l'oreille externe, au niveau du pavillon et du conduit auditif externe, et dans les trompes d'Eustache.

Selon un mode de réalisation de l'invention, le sujet auquel la composition telle que décrite ci-dessus est administrée est un animal, de préférence un mammifère, encore plus préférentiellement un Homme. Selon un mode de réalisation de l'invention, la composition est administrée à un animal domestique.

Selon un mode de réalisation de l'invention, le sujet auquel la composition telle que décrite ci-dessus est administrée souffre d'arthrose. Selon un autre mode de réalisation de l'invention, ledit sujet présente une prédisposition génétique à l'arthrose. Selon un autre mode de réalisation de l'invention, ledit sujet présente une prédisposition non-génétique à l'arthrose, sélectionnée parmi le groupe comprenant l'âge, tel que par exemple les personnes âgées de plus de 40 ans, de préférence de plus de 50 ans, encore plus préférentiellement de plus de 65 ans ; le surpoids ; la ménopause ; la survenue d'autres rhumatismes ; des traumatismes importants ou faibles à répétition ; l'anomalie de position de l'articulation, telle que, par exemple, une scoliose ou une malformation de la hanche ; la survenue d'autres maladies osseuses ou articulaires localisées, telles que, par exemple, des séquelles d'arthrite, des séquelles de fracture ou une maladie de Paget.

Selon un mode de réalisation de l'invention, la composition est administrée au moins une fois par jour, de préférence une fois par jour, encore plus préférentiellement une fois par jour le matin. Selon un autre mode de réalisation, la composition est administrée deux fois par jour, de préférence le matin et à midi. Selon un autre mode de réalisation, la composition est administrée trois fois par jour, de préférence le matin, à midi et le soir.

Selon un mode de réalisation de l'invention, la composition est administrée pendant au moins une semaine, de préférence au moins un mois, plus préférentiellement pendant au moins 6 mois.

Les exemples qui suivent montrent des modes de réalisation particuliers de l'invention, qui illustrent non limitativement l'invention.

### EXEMPLES

### Exemple 1

Une personne souffrant de rhumatismes des genoux a pris quotidiennement pendant plusieurs mois une gélule contenant de la Glucosamine, de la Chondroïtine et de la Vitamine C.

Elle a constaté que la douleur au niveau des genoux était nettement améliorée mais que la sensation de raideur articulaire n'était que très légèrement atténuée.

Cette personne a ensuite pris simultanément ces mêmes gélules et des comprimés de vitamine D sous forme de Cholécalciférol (25 µg).

Elle a constaté après quelques semaines une amélioration importante de la sensation de raideur au niveau des genoux. Cette nette amélioration s'est poursuivie au cours des semaines suivantes.

### Exemple 2

Cinq personnes souffrant de rhumatismes ont pris simultanément des comprimés contenant de la Glucosamine, de la Chondroïtine et de la Vitamine C et des comprimés de vitamine D sous forme de Cholécalciférol pendant plusieurs semaines: ces cinq personnes ont également observé une nette amélioration des raideurs articulaires.

### Exemple 3

Une personne souffrant de rhumatismes des genoux a pris quotidiennement pendant plusieurs mois une gélule contenant la composition 1.

Elle a constaté que la douleur au niveau des genoux et la sensation de raideur articulaire étaient nettement améliorées.

### Exemple 4

Des exemples de formulation sont, par comprimé:

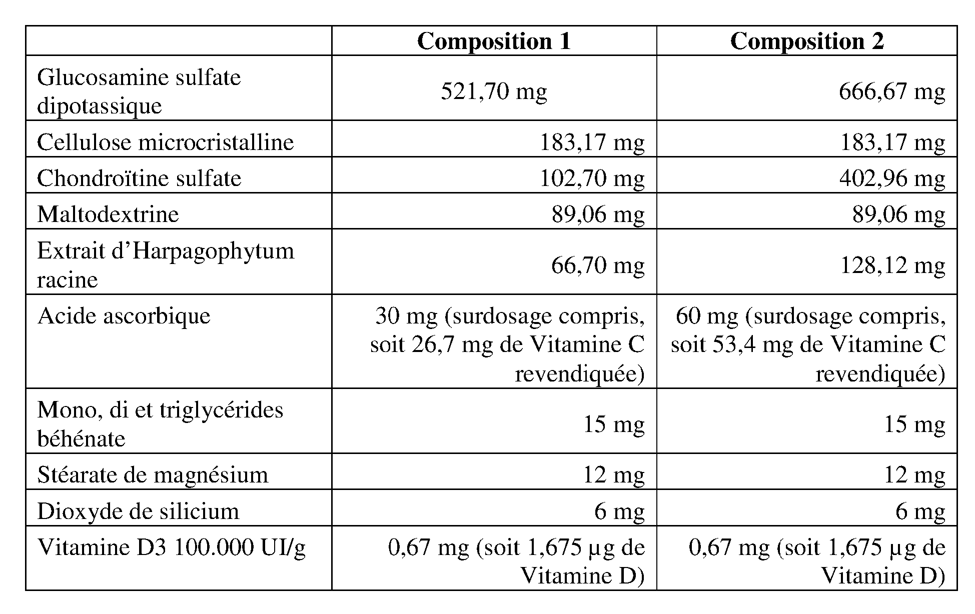

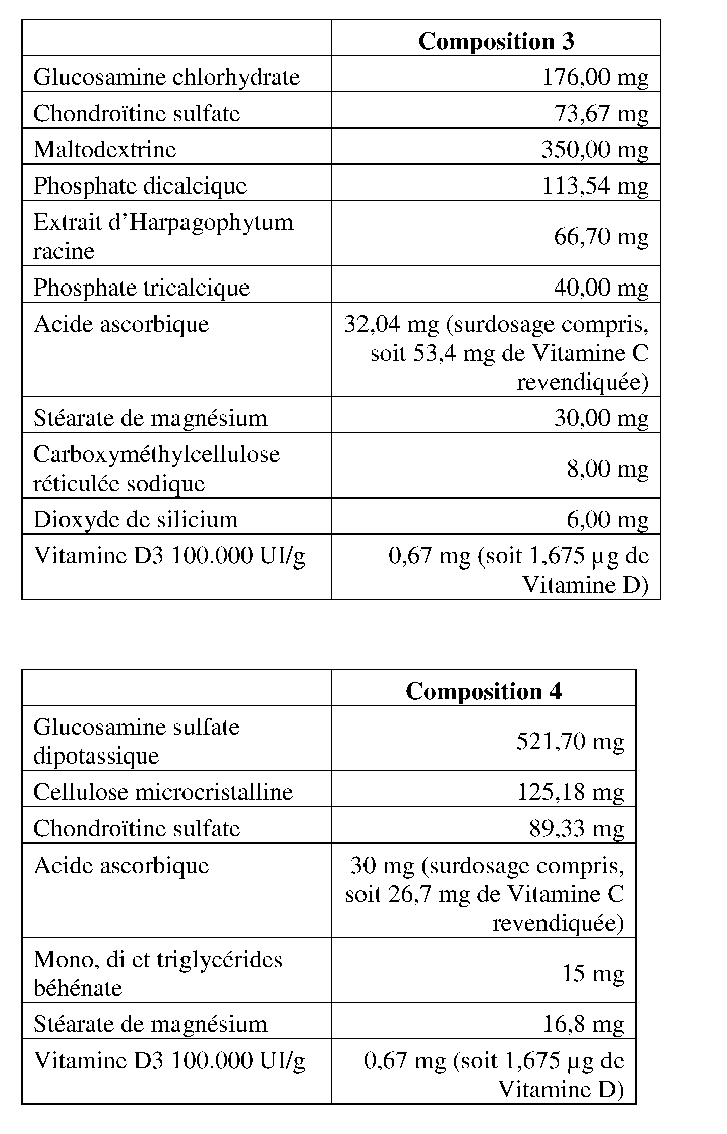

Ces compositions sont utilisées à la dose de 3 comprimés par jour.

## Revendications

1. Composition comprenant
- de la vitamine C,
- de la vitamine D, et
- au moins un élément chondroprotecteur sélectionné parmi le groupe comprenant la glucosamine et ses dérivés et la chondroïtine sulfate et ses dérivés,
ladite composition ne comprenant pas d'acide hyaluronique ou de collagène.

2. Composition selon la revendication **1, caractérisée en ce qu'**elle comprend un dérivé de glucosamine, de préférence sélectionné parmi le groupe comprenant la glucosamine sulfate, la glucosamine chlorhydrate et la N-glucosamine.

3. Composition selon l'une quelconque des revendications **1** ou **2, caractérisée en ce que** ladite composition est telle que la quantité de glucosamine ou de l'un de ses dérivés administrée par jour varie de 100 à 3000 mg/jour, de préférence de 500 à 2000 mg/jour, plus préférentiellement de 1000 à 1500 mg/jour, encore plus préférentiellement est d'environ 1200 mg/jour ; ou que ladite composition comprend de 50 à 3000 mg de glucosamine ou de l'un de ses dérivés, de préférence de 100 à 2000 mg, plus préférentiellement de 200 à 1750 mg, encore plus préférentiellement de 400 à 1500 mg, encore plus préférentiellement de 600 à 1200 mg, encore plus préférentiellement comprend environ 1200 mg de glucosamine ou de l'un de ses dérivés.

4. Composition selon l'une quelconque des revendications **1** à **3, caractérisée en ce qu'**elle comprend de la chondroïtine sulfate.

5. Composition selon l'une quelconque des revendications **1** à **4, caractérisée en ce que** ladite composition est telle que la quantité de chondroïtine sulfate administrée par jour varie de 100 à 3000 mg/jour, de préférence de 300 à 2000 mg/jour, plus préférentiellement de 1000 à 1500 mg/jour, encore plus préférentiellement est d'environ 1200 mg/jour ; ou que ladite composition comprend de 50 à 3000 mg de chondroïtine sulfate, de préférence de 100 à 2000 mg, plus préférentiellement de 200 à 1750 mg, encore plus préférentiellement de 400 à 1500 mg, encore plus préférentiellement de 600 à 1200 mg, encore plus préférentiellement comprend environ 1200 mg de chondroïtine sulfate.

6. Composition selon l'une quelconque des revendications **1** à **5, caractérisée en ce que** la vitamine C est sélectionnée parmi le groupe comprenant l'acide L-ascorbique, le L-ascorbate de sodium, le L-ascorbate de calcium, le L-ascorbate de potassium, le 6-palmitate de L-ascorbyl, le L-ascorbate de magnésium et le L-ascorbate de zinc.

7. Composition selon l'une quelconque des revendications **1** à **6, caractérisée en ce que** ladite composition est telle que la quantité de vitamine C administrée par jour varie de 10 à 2000 mg/jour, de préférence de 90 à 1000 mg/jour, plus préférentiellement de 150 à 500 mg/jour, encore plus préférentiellement de 160 à 250 mg/jour, encore plus préférentiellement est d'environ 180 mg/jour; ou que ladite composition comprend de 30 à 2000 mg de vitamine C, de préférence de 60 à 1000 mg, plus préférentiellement de 90 à 500 mg, encore plus préférentiellement de 180 à 250 mg, encore plus préférentiellement comprend environ 180 mg de vitamine C.

8. Composition selon l'une quelconque des revendications **1** à **7, caractérisée en ce que** la vitamine D est sélectionnée parmi le groupe comprenant le cholécalciférol, l'ergocalciférol et leurs dérivés conservant la même activité.

9. Composition selon l'une quelconque des revendications **1** à **8, caractérisée en ce que** ladite composition est telle que la quantité de vitamine D administrée par jour varie de 0.5 à 50 µg/jour, de préférence de 1 à 25 µg/jour, plus préférentiellement de 2.5 à 15 µg/jour, encore plus préférentiellement de 5 à 10 µg/jour, encore plus préférentiellement est d'environ 5 µg/jour; ou que ladite composition comprend de 0.5 à 50 µg de vitamine D, de préférence de 1 à 25 µg, plus préférentiellement de 2.5 à 15 µg, encore plus préférentiellement de 5 à 10 µg, encore plus préférentiellement est d'environ 5 µg de vitamine D.

10. Composition selon l'une quelconque des revendications **1** à **9, caractérisée en ce qu'**elle est un produit nutraceutique, un produit alimentaire, une boisson, un complément alimentaire, un alicament, un produit cosmétique, un produit d'alimentation vétérinaire ou un médicament.

11. Composition selon l'une quelconque des revendications **1** à **10, caractérisée en ce qu'**elle peut être administrée par voie orale, par voie buccale ou par voie transcutanée.

12. Composition selon l'une quelconque des revendications **1** à **11, caractérisée en ce qu'**elle est sous la forme de granules, comprimés, capsules souples, poudres, solutions pour injection, crèmes, gels, émulsions, onguents, lavements, suspensions, sirops, ampoules, inhalateurs, sprays pour la bouche, injections, gouttes, suppositoires, patches, patchs transdermiques, pâtes, pommades, gommes à mâcher, poudres à dissoudre, solutions ou gélules.

13. Composition selon l'une quelconque des revendications **1** à **12, caractérisée en ce qu'**elle est utile pour réduire la dégradation du cartilage et/ou accélérer sa régénération chez un sujet.

14. Composition selon l'une quelconque des revendications **1** à **12,** pour le traitement de la douleur articulaire.

15. Composition selon l'une quelconque des revendications **1** à **12** pour le traitement des rhumatismes ou de l'arthrose.
